# EUROPÄISCHE PATENTSCHRIFT

(11) **EP 0 814 656 B1**
(45) Veröffentlichungstag und Bekanntmachung des Hinweises auf die Patenterteilung: **12.05.1999**
(21) Anmeldenummer: 96901743.3
(22) Anmeldetag: 19.01.1996
(51) Int. Cl.: A01M 1/20, A61L 9/03

(54) **VERDAMPFERVORRICHTUNG**
EVAPORATOR
EVAPORATEUR

(30) Priorität: 20.03.1995 DE 29504734 U
(43) Veröffentlichungstag der Anmeldung: 07.01.1998
(73) Patentinhaber: PERYCUT-CHEMIE A.G., 8053 Zürich (CH)
(72) Erfinder: BENCSITS, Franz, CH-8053 Zürich (CH)
(74) Vertreter: Grünecker, Kinkeldey, Stockmair & Schwanhäusser Anwaltssozietät
(86) Internationale Anmeldenummer: EP9600229
(87) Internationale Veröffentlichungsnummer: WO9628969

(56) Entgegenhaltungen:
- EP-A- 0 249 926
- DE-A- 3 737 272
- DE-U- 9 014 309
- GB-A- 2 192 337
- US-A- 2 616 024

## Beschreibung

Die Erfindung betrifft eine Verdampfervorrichtung zum Verdampfen von leichtflüchtigen Flüssigkeiten, insbesondere von flüssigen Insektenvernichtungsmitteln, mit einem Behälter für die Flüssigkeit, einem die Flüssigkeit aufsaugenden Docht und einem dem Docht zugeordneten Heizelement.

Eine solche Verdampfervorrichtung ist aus der DE 37 37 272 C2 bekannt. Bei dieser vorbekannten Verdampfervorrichtung ist in einem Gehäuse ein Behälter angeordnet, der von unten in das Gehäuse eingeführt ist und in diesem beispielsweise durch Verschrauben befestigt werden kann. In dem Behälter ist eine leichtflüchtige Flüssigkeit, insbesondere ein flüssiges Insektenvernichtungsmittel, eingefüllt. Um die Flüssigkeit durch Erwärmen zu verdampfen, ist ein Docht durch eine obere Öffnung des Behälters eingeführt, wobei ein oberes Ende des Dochtes aus dem Behälter vorsteht, während sein unteres Ende in die Flüssigkeit eingetaucht ist. Um das obere Ende des Dochtes ist ein den Docht umgebendes Heizelement angeordnet, das einen oberen Abschnitt des Dochtes zum Verdampfen der aus dem Behälter aufgesaugten Flüssigkeit erwärmt.

Nachteilig bei der vorbekannten Verdampfervorrichtung ist, daß ein Nachfüllen des Behälters relativ schwierig ist, da der Behälter zusammen mit dem Docht aus dem Gehäuse entnommen werden muß. Weiterhin muß der Docht aus dem oberen Ende des Behälters entfernt werden, um weitere Flüssigkeit in dem Behälter einzugießen. Dazu kann der Docht beispielsweise in einem separaten, mit dem oberen Ende des Behälters verschraubbaren Halteraufsatz befestigt sein. Analog ist nach Befüllen des Behälters das erneute Einsetzen von Docht und vom Behälter in das Gehäuse relativ aufwendig.

Weiterhin ist von Nachteil, daß der Docht mit seinem unteren Ende genau plaziert werden muß, um die im Behälter enthaltene Flüssigkeit vollständig aufzusaugen. Ist das untere Ende des Dochtes vom Behälterboden beabstandet, bleibt ein nicht nutzbarer Rest der Flüssigkeit im Behälter zurück.

Der Erfindung liegt daher die Aufgabe zugrunde, eine Verdampfervorrichtung der eingangs genannten Art dahingehend zu verbessern, daß die Handhabung vereinfacht und gleichzeitig sichergestellt ist, daß die im Behälter enthaltene Flüssigkeit vollständig verdampfbar ist.

Diese Aufgabe wird im Zusammenhang mit den Merkmalen des Oberbegriffs des Anspruchs 1 dadurch gelöst, daß der Docht beabstandet zum Behälter angeordnet und mit diesem über eine Versorgungsleitung zur Zufuhr der Flüssigkeit verbunden ist.

Auf diese Weise ist der Behälter unabhängig vom Docht handhabbar bzw. befüllbar. Der Behälter kann ohne Entnahme aus einem entsprechenden Gehäuse befüllt werden, da beispielsweise im Gegensatz zur DE 37 37 272 C2 kein Docht aus einem oberen Behälterende hervorsteht. Weiterhin entfällt die Notwendigkeit, den Docht im Behälter so zu positionieren, daß die Flüssigkeit vollständig aufgesaugt werden kann. Statt dessen wird dem Docht über die Versorgungsleitung die Flüssigkeit bis zur vollständigen Entleerung des Behälters zugeführt. Ein weiterer Vorteil ist, daß die erfindungsgemäße Verdampfervorrichtung schneller einsetzbar ist, da die Flüssigkeit nicht entlang des gesamten Dochtes durch Kapillarwirkung oder ähnliches in Richtung Heizelement transportiert werden muß. Über die Versorgungsleitung fließt die Flüssigkeit relativ schnell direkt zum Docht, der dabei im Vergleich zur DE 37 37 272 C2 mit vergleichsweise geringen Abmessungen ausgebildet ist.

Um die vollständige Entleerung des Behälters in einfachster Weise zu sichern, ist der Doch< unterhalb des Behälters angeordnet. Auf diese Weise kann die Flüssigkeit selbsttätig zum Docht ohne weitere Hilfsmittel, wie beispielsweise Pumpen oder dergleichen fließen.

Der Docht kann in ansich bekannter Weise aus verschiedenen Materialien hergestellt sein. Solche Materialien sind beispielsweise verschiedene Fasern, wie Filz, Baumwolle oder dergleichen. Vorzugsweise ist der Docht aus einem anorganischen, porösen Material gebildet. Zu diesen Materialen zählen Keramik, Glasfasern, Gips, Bentonit, Kaolin, Talk, Diatomeenerde, Perlit usw. Der Docht kann aus einem Pulver eines oder auch mehrerer dieser Materialien gepreßt sein.

Um den Docht einfach herstellen zu können und gleichzeitig relativ große Oberflächen zum Verdampfen der Flüssigkeit aufzuweisen, ist der Docht vorzugsweise als im wesentlichen quaderförmiger Verdampferkörper ausgebildet.

In diesem Zusammenhang ist es weiterhin von Vorteil, wenn das Heizelement einer einer verdampften Flüssigkeit abgebenden Oberseite des Verdampferkörpers gegenüberliegenden Unterseite zugeordnet ist und die Versorgungsleitung an einer Seitenfläche des Verdampferkörpers angeschlossen ist. Dadurch wird dem Verdampferköper über eine Seitenfläche Flüssigkeit zugeführt, die Unterseite erhitzt und über die Oberseite die verdampfte Flüssigkeit abgegeben. Oberseite und Unterseite weisen dabei in der Regel im Vergleich zur Seitenfläche größere Abmessungen auf, so daß eine schnelle Erwärmung und ein schnelles Verdampfen der Flüssigkeit einfach möglich ist. Gleichzeitig kann der Verdampferkörper relativ kompakt sein.

Um die Versorgungsleitung in einfacher Weise an der Seitenfläche des Verdampferkörpers anschließen zu können, weist dieser einen in ein erstes Ende der Versorgungsleitung einsteckbaren Vorsprung auf. Vorzugsweise ist der Vorsprung aus dem gleichen Material wie der Verdampferkörper gebildet.

Um den Verdampferkörper in einfacher Weise zu halten, ist dieser zumindest mit seiner Unterseite auf einem wärmeleitenden Tragblech gelagert, an dem das Heizelement angeordnet ist. Durch die Wärmeleitung des Tragbleches kann das Heizelement kleiner als die Unterseite des Verdampferkörpers ausgebildet sein. Die vom Heizelement erzeugte Wärme wird durch das Tragblech auf die gesamte Unterseite des Verdampferkörpers übertragen. Dadurch kann eine gleichmäßige Erwärmung und folglich eine gleichmäßige Verdampfung von der gesamten Oberseite des Verdampferkörpers erfolgen.

In diesem Zusammenhang ist es günstig, wenn das Heizelement auf einer dem Verdampferkörper gegenüberliegenden Unterseite des Tragbleches angeordnet ist. Dadurch erfolgt die Wärmeübertragung direkt durch das Tragblech auf die Unterseite des Verdampferkörpers.

Behälter und Verdampferkörper können bei der erfindungsgemäßen Verdampfervorrichtung entsprechend zur DE 37 37 272 C2 in einem Gehäuse angeordnet sein. weiterhin kann der Behälter lösbar am Gehäuse befestigt sein. Um ein einfaches Befestigen und Lösen des Behälters zu erreichen, stehen vom Behälter Rastelemente ab und am Gehäuse sind mit diesem verrastbare Gegenrastelemente ausgebildet.

Rastelemente und Gegenrastelemente können in mannigfacher Weise ausgebildet sein. Bevorzugt ist eine Ausbildung der Rastelemente als von einer Unterseite des Behälters abstehende Rasthaken, welche in als Gegenrastelemente in einer am Gehäuse ausgebildeten Aufnahmefläche für die Behälterunterseite angeordnete Rastöffnungen eingreifen. Auf diese Weise wird der Behälter einfach von oben auf die Aufnahmefläche aufgesetzt und eingerastet.

Um die Versorgungsleitung am Behälter an einer günstigen Stelle und einfach befestigen zu können, steht ein Anschlußzapfen von der Behälterunterseite ab, auf welchen ein zweites Ende der Versorgungsleitung aufsteckbar ist, wobei eine dem Anschlußzapfen entsprechende Öffnung in der Aufnahmefläche ausgebildet ist. Der Anschlußzapfen kann so beim Aufrasten des Behälters auf die Aufnahmefläche in die zugeordnete Öffnung eingeführt werden. Unterhalb dieser Aufnahmefläche und gegebenenfalls zusätzlich seitlich versetzt zum Behälter kann der Verdampferkörper angeordnet sein, wobei zwischen Anschlußzapfen und Vorsprung des Verdampferkörpers die Versorgungsleitung verläuft.

Um die Zuordnung von Aufnahmefläche und Behälter zu vereinfachen, weist die Aufnahmefläche einen in Richtung Behälter nach oben abstehenden Umfangsrand auf, welcher zumindest entlang einer Seite des Behälters eine Anlagewand bildet, die eine im wesentlichen einer Behälterhöhe entsprechende Höhe aufweist. Der Behälter liegt dabei mit seiner der Anlagewand zugeordneten Seite an dieser an. Die Anlagewand kann eben oder gekrümmt ausgebildet sein. Vorzugsweise ist der Behälter im wesentlichen kastenförmig.

Um den Behälter in einfacher Weise befüllen zu können, weist dieser an seinem oberen Ende eine von einem im wesentlichen horizontalen Rand umgebene Einfüllöffnung auf, wobei ein auf die Einfüllöffnung aufschiebbarer Deckel eine zum Rand komplementäre Aufschiebnut aufweist. Durch diese spezielle Konstruktion des Deckels ist ein zufälliges Öffnen, beispielsweise durch Kinder, verhindert, so daß diese nicht in Kontakt mit der Flüssigkeit kommen.

Der Deckel kann bei einem Ausführungsbeispiel der Erfindung einen in Richtung Anlagewand abstehenden Flansch aufweisen, der bei auf der Einfüllöffnung aufgeschobenen Deckel in eine am oberen Ende der Anlagewand gebildeten Durchbruch eingeführt ist. Dieser Flansch kann als Handhabe zum Lösen des Deckels verwendet werden.

Dabei ist es weiterhin von Vorteil, wenn im Durchbruch zwei Rastschrägen von der Anlagewand abstehen und der Deckelflansch komplementäre, die Rastschrägen hintergreifende Rastvorsprünge aufweist. Damit ist das Abnehmen des Deckels soweit erschwert, daß zunindest kleinere Kinder den Deckel nicht vom Behälter entfernen können. Ebenso ist ein zufälliges Entfernen des Deckels nicht möglich.

Um den Deckel dennoch relativ einfach vom Behälter abschieben zu können, stehen die Rastvorsprunge seitlich und horizontal vom Deckelflansch ab. Ein vorderes Ende des Deckelflansches kann zum Öffnen des Deckels untergriffen werden, wodurch der Rasteingriff zwischen Rastvorsprung und Rastschräge aufgehoben ist und der Deckel entlang des Öffnungsrandes abgeschoben werden kann.

Um ein nach außen geschlossenes Gehäuse zu erhalten und gleichzeitig bei der Herstellung der Verdampfervorrichtung in einfacher Weise alle notwendigen Teile in das Gehäuse einsetzen zu können, weist das Gehäuse ein Gehäuseoberteil und ein Gehäuseunterteil auf, wobei aufeinander zuweisende Wandabschnitte von Gehäuseober- und Gehäuseunterseite einander hintergreifen.

Um in diesem Zusammenhang auf zusätzliche Einrichtungen zur Verbindung der beiden Gehäuseteile verzichten zu können, wie Schrauben oder dergleichen, weisen im Gehäuseunterteil und -oberteil komplementär zueinander ausgebildete Rasteinrichtungen auf. Diese sind bei zusammengesetztem Gehäuse miteinander in Eingriff.

Um die Verdampfervorrichtung in einfacher Weise an einer Steckdose anschließen zu können, ist am Gehäuse ein Steckerteil angeordnet. Dabei erweist es sich als günstig, wenn das Steckerbauteil im Gehäuseunter- und/oder Gehäuseoberteil um einen Winkel von zumindest 90° drehbar gelagert ist. Auf diese Weise kann die Verdampfervorrichtung sowohl für Steckdosen mit horizontal als auch vertikal nebeneinander bzw. übereinander angeordneten Steckkontakten verwendet werden, ohne daß Flüssigkeit aus dem Behälter austritt. Der Drehwinkel kann durch entsprechende Anschläge am Steckerbauteil und/oder am Gehäuse begrenzt werden, wobei ein Anschlag der horizontalen und ein Anschlag der vertikalen Anordung der Steckkontakte der Steckdose entspricht.

Um die Verdampfervorrichtung einfach betätigen zu können und gleichzeitig deren Betriebszustand feststellen zu können, erweist es sich weiterhin als günstig, wenn der Steckerbauteil über einen Schalter und eine Anzeigelampe mit dem Heizelement elektrisch verbunden ist. Der Schalter kann beispielsweise ein zwei verschiedene Betriebszustände der Verdampfervorrichtung steuernder Kippschalter sein. Die Betriebszustände unterscheiden sich dabei in der Regel durch die Heizleistung des Heizelementes. Als Anzeigelampe wird vorzugsweise eine LED-Anzeigelampe verwendet. Diese kann an im wesentlichen jeder beliebigen Stelle des Gehäuses angeordnet sein. Ist der Behälter beispielsweise aus einem durchsichtigen Material gefertigt, kann die Anzeigelampe auch in der Aufnahmefläche angeordnet sein, so daß sie durch den Behälter sichtbar ist.

Gehäuse und Behälter sind vorzugsweise aus einem elektrisch isolierenden Material, wie Kunststoff oder dergleichen, hergestellt.

Im folgenden wird ein vorteilhaftes Ausführungsbeispiel der Erfindung anhand der in der Zeichnung beigefügten Figuren näher erläutert und beschrieben.

Es zeigen:
- Fig. 1: eine teilweise gebrochene Seitenansicht einer Verdampfervorrichtung gemäß der Erfindung;
- Fig. 2: eine Draufsicht auf die Verdampfereinrichtung nach Fig. 1 mit abgenommenem Behälter;
- Fig. 3: eine Seitenansicht eines von der Verdampfervorrichtung nach Fig. 1 abgenommenen Behälters;
- Fig. 4: eine Draufsicht auf einen auf den Behälter aufschiebbaren Deckel; und
- Fig. 5: eine Draufsicht auf ein in Fig. 1 dargestelltes Tragblech für einen Verdampferkörper.

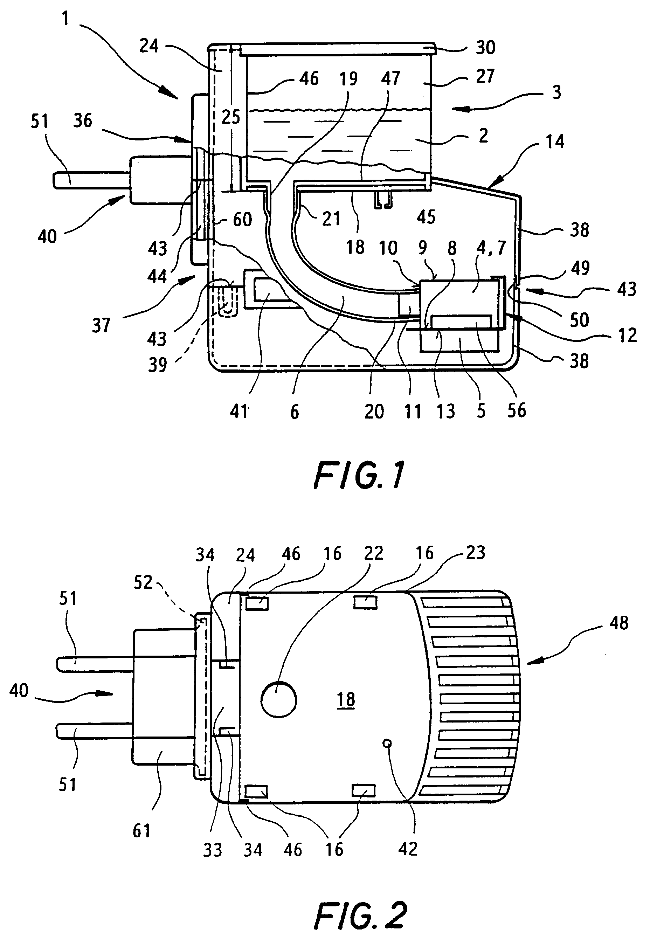

Bei der Seitenansicht einer Verdampfervorrichtung 1 nach Fig. 1 weist diese einen in Flüssigkeit 2 enthaltenden Behälter 3 und ein Gehäuse 14 auf. Der Behälter 3 ist von oben auf eine ebene Aufnahmefläche 18 des Gehäuses 14 aufgesetzt. Diese weist einen zum Behälterquerschnitt entsprechenden Umriß auf, siehe auch Fig. 2. Der Behälter ist an seinem oberen Ende durch einen Deckel 30 verschlossen, der in Fig. 1 von rechts nach links auf dem Behälter aufschiebbar ist. Der Deckel verschließt eine am oberen Ende 27 des Behälters gebildete Einfüllöffnung und erstreckt sich bis zu einer Anlagewand 24. Diese bildet im wesentlichen einen Teil eines die Aufnahmefläche 18 umgebenden Randes und erstreckt sich senkrecht zur Aufnahmefläche 18. Der Behälter 3 ist von oben auf die Aufnahmefläche 18 aufsetzbar, wobei er von dem Rand 23, siehe Fig. 2, und von an der Anlagewand 24 beidseitig abstehenden Wandvorsprüngen 46, siehe auch Fig. 2, seitlich aufgenommen ist.

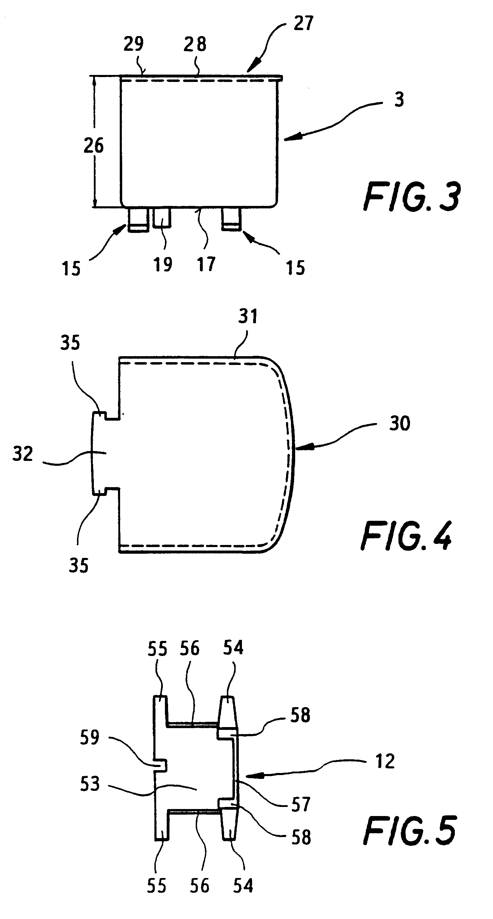

Die Anlagewand 24 weist eine Höhe 25 auf, die im wesentlichen einer Behälterhöhe 26 nach Fig. 3 entspricht. Dabei ist die Wandhöhe um soviel länger als die Behälterhöhe ausgebildet, daß der Deckel 30 flächenbündig zum oberen Ende der Anlagewand 24 angeordnet ist.

Im Behälterboden 47 ist ein Anschlußzapfen 19 ausgebildet, welcher im wesentlichen hohlzylinderförmig ist. Dieser erstreckt sich durch eine Öffnung 22 der Aufnahmefläche 18, siehe auch Fig. 2, bis in das Gehäuse 14. Auf den Anschlußzapfen 19 ist ein zweites Ende 21 einer Versorgungsleitung 16 aufgesteckt. Deren gegenüberliegendes, erstes Ende 20 ist auf einem zapfenartigen Vorsprung 11 eines als Docht wirkenden Verdampferkörpers 7 aufgesteckt. Der Verdampferkörper 7 ist quaderförmig mit Oberseite 9, Unterseite 8 und vier diese verbindenden Seitenflächen 10. Dabei entsteht der Vorsprung 11 von einer der Versorgungsleitung zuweisenden Seitenfläche 10 des Verdampferkörpers 7 ab.

Zur Halterung des Verdampferkörpers 7 im Gehäuse 14 ist ein Tragblech 12 angeordnet. Dieses weist eine im wesentlichen horizontale Tragfläche auf, auf der der Verdampferkörper 7 aufliegt. Seitlich ist der Verdampferkörper 7 von Seitenwänden 56 und an seiner dem Vorsprung 11 gegenüberliegenden Rückseite von einer Rückwand gehalten, welche an ihrem oberen Ende zumindest zwei auf die Oberseite 9 des Verdampferkörpers 7 umgefalzte Haltelaschen aufweist. Zur genaueren Darstellung des Tragbleches 12 wird auf Fig. 5 verwiesen.

Das Gehäuse 14 ist aus einem Gehäuseoberteil 36 und einem Gehäuseunterteil 37 zusammengesetzt. Die beiden Gehäuseteile treffen entlang einer Verbindungslinie 43 aufeinander. Im Bereich dieser Verbindungslinie stehen von aufeinander zuweisenden Wandabschnitten 38 der Gehäuseteile gegenüber der sonstigen Wandstärke dünnere Wandspitzen 49 bzw. 50 vor, die einander hintergreifen.

Im Bereich der Verbindungslinie 43 ist ein Schalter 41 angeordnet. Zu dessen Aufnahme und Befestigung sind in an sich bekannter Weise Ausnehmungen in den aufeinander zuweisenden Wandabschnitten der Gehäuseteile gebildet.

Zur Verbindung der beiden Gehäuseteile ist eine Rasteinrichtung 39 vorgesehen. Diese weist beispielsweise vom Gehäuseoberteil 36 vorstehende Rastlaschen und im Gehäuseunterteil 37 an dessen Innenseiten angeordnete Rastvorsprünge auf.

An einer Seite der Verdampfervorrichtung 1 ist ein Steckerteil 40 mit zwei Kontaktfingern 51 angeordnet. Das Steckerteil 40 ist in einer im wesentlichen kreisförmigen, durch beide Gehäuseteile jeweils zur Hälfte gebildeten Drehführung gehalten. Die Drehführung weist eine Drehnut 44 auf, in der das Steckerteil 40 drehbar gelagert ist. Zur Bildung der Drehführung steht vom Gehäuseunterteil 37 an dessen dem Steckerteil 40 zuweisenden Ende eine Vorderwand 60 nach oben ab. An dieser ist eine Hälfte der Drehführung mit entsprechender Drehnut 44 angeformt.

Im Bereich der Aufnahmefläche 18 ist ein LED-Halter 45 angeordnet. Dieser dient zur Halterung einer nicht dargestellten LED-Anzeigelampe, die durch eine entsprechende Öffnung in der Aufnahmefläche 18 durch den aus einem durchsichtigen Material hergestellten Behälter 3 sichtbar ist.

In Fig. 2 ist eine Draufsicht auf die Verdampfervorrichtung nach Fig. 1 dargestellt. Gleiche Bezugszeichen kennzeichnen gleiche Teile und werden auch teilweise erwähnt.

In Fig. 2 ist der Behälter 3 zur Vereinfachung nicht dargestellt. Dadurch ist die Aufnahmefläche 18 mit vier als Gegenrastelementen ausgebildeten Rastöffnungen 16, einer Öffnung 22 für den Anschlußzapfen 19 des Behälters 3, siehe Fig. 1, und eine Öffnung 42 für eine LED-Anzeigelampe sichtbar. Die Aufnahmefläche 18 weist einen im wesentlichen rechteckförmigen Umriß auf, wobei eine Anzahl von Lüftungsschlitzen 48 zugewandte Seite nach außen konvex gekrümmt ist. Die Aufnahmefläche 18 ist entlang ihres Umfanges von einem in Richtung Behälter nach oben überstehenden Umfangsrand 23 umrandet. Dieser bildet auf der dem Steckerteil 40 zuweisenden Seite die Anlagewand 24. Diese weist an ihren beiden Seiten in Richtung Aufnahmefläche 18 vorstehende Wandvorsprünge 46 auf, zwischen denen und dem nach oben vorstehenden Umfangsrand 23 der nicht dargestellte Behälter gehalten ist. An ihrem oberen Ende weist die Anlagewand 24 einen Durchbruch 33 auf. Dieser weist einen rechteckförmigen Umriß auf, wobei entlang der kürzeren Rechteckseiten rampenförmige Rastschrägen 34 gebildet sind. Diese weisen eine in Richtung Steckerteil 40 zunehmende Höhe auf.

Aus der der Auflagefläche 18 gegenüberliegenden Außenseite der Anlagewand 24 ist die mit der Nut 44 nach Fig. 1 ausgebildete Drehführung angeordnet. In dieser ist Steckerteil 40 mit einem radial abstehenden Randflansch 52 drehbar gelagert. Aus der Drehführung steht ein Einsteckteil 61 zum Einstecken in eine Steckdose des Steckerteils 40 über, wobei von dem Einsteckteil 61 zwei Kontaktfinger 51 vorstehen.

In Fig. 3 ist eine Seitenansicht des Behälters 3 dargestellt. Dieser weist eine Anzahl von einer Unterseite 17 des Behälterbodens vorstehende, als Rastelemente ausgebildete Rasthaken 15 und den Anschlußzapfen 19 auf. Behälterunterseite 17, Rasthaken 15 und Anschlußzapfen 19 sind so ausgebildet und angeordnet, daß sie entsprechend auf die Aufnahmefläche 18 aufsetzbar bzw. in die Rastöffnungen 16 bzw. Öffung 22 einsetzbar sind.

Am oberen Ende 27 des Behälters 3 ist eine Einfüllöffnung 29 gebildet, die von einem nach außen und horizontal vom Behälter abstehenden Rand 28 umrandet ist.

In Fig. 4 ist eine Draufsicht auf den Deckel 30 dargestellt. Dieser weist eine dem Umriß des oberen Endes 27 des Behälters 3 entsprechende Form auf. Der Deckel weist entlang seines Randes eine Aufschiebenut 31 auf. Diese ist dadurch gebildet, daß der Behälterrand zuerst nach unten und dann nach innen abgeknickt ist, wobei die Nut zwischen dem nach innen abgeknickten Teil und der Oberseite des Deckels 30 gebildet ist. Auf seiner der Anlagewand 24 nach Figuren 1 bzw.2 zuweisenden Seite weist der Deckel 30 keine Aufschiebenut 31 auf. Statt dessen ist an dieser Seite ein Flansch 32 ausgebildet, von dem seitlich jeweils ein Rastvorsprung 35 absteht. Diese Rastvorsprünge 35 gleiten beim Aufschieben des Deckels 30 auf dem Behälter 3 über die Rastschrägen 34 nach Fig. 2. Bei vollständig aufgeschobenem Deckel hintergreifen die Rastvorsprünge 35 die Rastschrägen 34, so daß Deckel 30 in seiner Schließstellung gesichert ist.

In Fig. 5 ist Tragblech 12 nach Fig. 1 ohne Verdampferkörper 7 dargestellt. Das Tragblech 12 ist aus einem wärmeleitenden Material gebildet, wie beispielsweise Stahlblech oder dergleichen. Es weist eine ebene Tragfläche 53 auf, auf welcher der Verdampferkörper auflegbar ist. Zum seitlichen Halten des Verdampferkörpers stehen von der Tragfläche 53 zwei Seitenwände 56, siehe auch Fig. 1, nach oben ab. Zum Halten eines hinteren Endes des Verdampferkörpers steht weiterhin eine Rückwand 57 von der Tragfläche 53 nach oben ab. Seitenwände 56 und Rückwand 57 bilden gleichzeitig einen Rand der Tragfläche 53. In der Regel weist die Rückwand 57 eine größere Höhe als die Seitenwände 56 auf. An ihrem oberen Ende weist die Rückwand 57 zwei im wesentlichen parallel zur Tragfläche 53 angeordnete Haltelaschen 58 auf. Diese liegen nach Fig. 1 auf der Oberseite 9 des Verdampferkörpers 7 auf.

In Verlängerung der Rückwand 57 und an beiden Enden der Seitenwände 56 stehen Auflagefinger 54 bzw. 55 von der Tragfläche 53 ab. Diese dienen zur Befestigung des Tragbleches 12 im Gehäuse 14 nach Fig. 1. Die Befestigung kann dabei so erfolgen, daß jeweils vier von oben auf den Auflagefingern 54 bzw. 55 aufstehende Vorsprünge im Gehäuseoberteil und entsprechend vier von unten an den Auflagefingern aufstehende Vorsprünge vom Gehäuseunterteil abstehen, welche bei miteinander verrasteten Gehäuseteilen die Auflagefinger zwischen sich klemmen.

Um das Heizelement 5 nach Fig. 1 auf der Unterseite der Tragfläche 53 zu halten, ist unter Bildung einer Ausnehmung 59 ein vorderer Abschnitt der Tragfläche 53 nach unten ungefalzt und untergreift das Heizelement 5, wodurch es an Unterseite 13 des Tragbleches 12, siehe Fig. 1, angepreßt ist.

## Patentansprüche

1. Verdampfervorrichtung (1) zum Verdampfen von leichtflüchtigen Flüssigkeiten (2), insbesondere von flüssigen Insektenvernichtungsmitteln, mit einem Behälter (3) für die Flüssigkeit (2), einem die Flüssigkeit aufsaugenden Docht (4) und einem dem Docht zugeordneten Heizelement (5), **dadurch gekennzeichnet**, daß der Docht (4) beabstandet zum Behälter (3) angeordnet und mit diesem über eine Versorgungsleitung (6) zur Zufuhr der Flüssigkeit (2) verbunden ist.

2. Verdampfervorrichtung nach Anspruch 1, **dadurch gekennzeichnet**, daß der Docht (4) unterhalb des Behälters (3) angeordnet ist.

3. Verdampfervorrichtung nach Anspruch 1 oder 2, **dadurch gekennzeichnet**, daß der Docht (4) aus einem anorganischen, porösen Material gebildet ist.

4. Verdampfervorrichtung nach Anspruch 3, **dadurch gekennzeichnet**, daß der Docht (4) aus einem im wesentlichen quaderförmigen Verdampferkörper (7) gebildet ist.

5. Verdampfervorrichtung nach Anspruch 4, **dadurch gekennzeichnet**, daß das Heizelement (5) einer einer die verdampfte Flüssigkeit (2) abgebenden Oberseite (9) gegenüberliegenden Unterseite (8) des Verdampferkörpers (7) zugeordnet ist und die Versorgungsleitung (6) an einer Seitenfläche (10) des Verdampferkörpers (7) angeschlossen ist.

6. Verdampfervorrichtung nach Anspruch 5, **dadurch gekennzeichnet**, daß die Seitenfläche (10) einen in ein erstes Ende (20) der Versorgungsleitung (6) einsteckbaren Vorsprung (11) aufweist.

7. Verdampfervorrichtung nach Anspruch 4, **dadurch gekennzeichnet**, daß der Verdampferkörper (7) zumindest mit seiner Unterseite (8) auf einem wärmeleitenden Tragblech (12) gelagert ist, an dem das Heizelement (5) angeordnet ist.

8. Verdampfervorrichtung nach Anspruch 4, **dadurch gekennzeichnet**, daß das Heizelement (5) auf einer dem Verdampferkörper (7) gegenüberliegenden Unterseite (13) des Tragbleches (12) angeordnet ist.

9. Verdampfervorrichtung mit einem Behälter (3), Verdampferkörper (7), Tragblech (12) und Heizelement (5) aufnehmenden Gehäuse (14), wobei der Behälter (3) lösbar am Gehäuse befestigt ist, nach Anspruch 7, **dadurch gekennzeichnet**, daß vom Behälter (3) Rastelemente (15) abstehen und mit diesen verrastbare Gegenrastelemente (16) am Gehäuse (14) ausgebildet sind.

10. Verdampfervorrichtung nach Anspruch 9, **dadurch gekennzeichnet**, daß die Rastelemente (15) als von einer Unterseite (17) des Behälters (3) abstehende Rasthaken ausgebildet sind und die Gegenrastelemente (16) in einer am Gehäuse (14) ausgebildeten Aufnahmefläche (18) für die Behälterunterseite (17) angeordnete Rastöffnungen sind.

11. Verdampfervorrichtung nach Anspruch 10, **dadurch gekennzeichnet**, daß ein Anschlußzapfen (19) von der Behälterunterseite (17) absteht, auf welchem ein zweites Ende (21) der Versorgungsleitung (6) aufsteckbar ist, wobei eine dem Anschlußzapfen (19) entsprechende Öffnung (22) in der Aufnahmefläche (18) ausgebildet ist.

12. Verdampfervorrichtung nach Anspruch 10, **dadurch gekennzeichnet**, daß die Aufnahmefläche (18) einen in Richtung Behälter (3) nach oben abstehenden Umfangsrand (23) aufweist, welcher zumindest entlang einer Seite des Behälters (3) eine Anlagewand (24) bildet, die eine im wesentlichen einer Behälterhöhe (26) entsprechende Höhe (25) aufweist.

13. Verdampfervorrichtung nach wenigstens einem der vorangehenden Ansprüche, **dadurch gekennzeichnet**, daß der Behälter (3) an seinem oberen Ende (27) eine von einem im wesentlichen horizontalen Rand (28) umgebende Einfüllöffnung (29) aufweist, wobei ein auf die Einfüllöffnung aufschiebbarer Deckel (30) eine zum Rand komplementäre Aufschiebnut (31) aufweist.

14. Verdampfervorrichtung nach Anspruch 13, **dadurch gekennzeichnet**, daß der Deckel (30) einen in Richtung Anlagewand (24) abstehenden Flansch aufweist, der bei auf der Einfüllöffnung (29) aufgeschobenem Deckel (30) in einen am oberen Ende der Anlagewand (24) gebildeten Durchbruch (23) eingeführt ist.

15. Verdampfervorrichtung nach Anspruch 14, **dadurch gekennzeichnet**, daß im Durchbruch (33) zwei Rastschrägen (34) von der Anlagewand (24) abstehen und der Deckelflansch (32) komplementäre, die Rastschrägen (34) hintergreifende Rastvorsprünge (35) aufweist.

16. Verdampfervorrichtung nach Anspruch 15, **dadurch gekennzeichnet**, daß die Rastvorsprünge (35) seitlich und horizontal vom Deckelflansch (32) abstehen.

17. Verdampfervorrichtung nach wenigstens einem der vorangehenden Ansprüche, **dadurch gekennzeichnet**, daß das Gehäuse (14) ein Gehäuseoberteil (36) und ein Gehäuseunterteil (37) aufweist, wobei aufeinanderzuweisende Wandabschnitte (38) von Gehäuseober- und Gehäuseunterteil (36, 37) einander hintergreifen.

18. Verdampfervorrichtung nach Anspruch 17, **dadurch gekennzeichnet**, daß Gehäuseober- und Gehäuseunterteil (36, 37) komplementär zueinander ausgebildete Rasteinrichtungen (39) aufweist.

19. Verdampfervorrichtung nach Anspruch 17, **dadurch gekennzeichnet**, daß ein Steckerbauteil (40) im Gehäuseoberteil (36) und/oder im Gehäuseunterteil (37) um einen Winkel von zumindest 90° drehbar gelagert ist.

20. Verdampfervorrichtung nach Anspruch 19, **dadurch gekennzeichnet**, daß das Steckerbauteil (40) über zumindest einen Schalter (41) und eine Anzeigelampe (42) mit dem Heizelement (5) elektrisch verbunden ist.

## Claims

1. Evaporator (1) for evaporating highly volatile liquids (2), in particular liquid insecticides, having a container (3) for the liquid (2), a wick (4) which absorbs the liquid and a heating element (5) associated with the wick, **characterized in** that the wick (4) is disposed at a distance from the container (3) and is connected to the latter by a supply line (6) for supplying the liquid (2).

2. Evaporator according to claim 1, **characterized in** that the wick (4) is disposed underneath the container (3).

3. Evaporator according to claim 1 or 2, **characterized in** that the wick (4) is formed from an inorganic, porous material.

4. Evaporator according to claim 3, **characterized in** that the wick (4) is formed from an evaporator body (7) of substantially parallelopiped form.

5. Evaporator according to claim 4, **characterized in** that the heating element (5) is associated with an underside (8) of the evaporator body (7) lying opposite a top side (9), which releases the evaporated liquid (2), and the supply line (6) is connected to a side surface (10) of the evaporator body (7).

6. Evaporator according to claim 5, **characterized in** that the side surface (10) has a projection (11), which is insertable into a first end (20) of the supply line (6).

7. Evaporator according to claim 4, **characterized in** that the evaporator body (7) is supported at least by its underside (8) on a heat-conducting supporting plate (12), against which the heating element (5) is disposed.

8. Evaporator according to claim 4, **characterized in** that the heating element (5) is disposed on an opposite underside (13) of the supporting plate (12) to the evaporator body (7).

9. Evaporator having a housing (14) receiving container (3), evaporator body (7), supporting plate (12) and heating element (5), the container (3) being fastened releasably to the housing, according to claim 7, **characterized in** that detent elements (15) protrude from the container (3) and are latchable with counterpart detent elements (16) formed on the housing (14).

10. Evaporator according to claim 9, **characterized in** that the detent elements (15) take the form of detent hooks, which protrude from an underside (17) of the container (3), and the counterpart detent elements (16) are detent openings disposed in a locating surface (18), which is formed for the container underside (17) on the housing (14).

11. Evaporator according to claim 10, **characterized in** that projecting from the container underside (17) is a connection shank (19), on which a second end (21) of the supply line (6) is mountable, an opening (22) which corresponds to the connection shank (19) being formed in the locating surface (18).

12. Evaporator according to claim 10, **characterized in** that the locating surface (18) has a peripheral edge (23), which projects upwards in the direction of the container (3) and which at least along one side of the container (3) forms a seating wall (24) of a height (25) corresponding substantially to a container height (26).

13. Evaporator according to at least one of the preceding claims, **characterized in** that the container (3) at its top end (27) has a charging opening (29) surrounded by a substantially horizontal rim (28), a lid (30) fittable onto the charging opening having a fitting groove (31) which is complementary to the rim.

14. Evaporator according to claim 13, **characterized in** that the lid (30) has a flange, which protrudes in the direction of the seating wall (24) and which, when the lid (30) is fitted on the charging opening (29), is introduced into a hole (23) formed at the top end of the locating wall (24).

15. Evaporator according to claim 14, **characterized in** that in the hole (33) two detent bevels (34) protrude from the seating wall (24) and the lid flange (32) has complementary detent projections (35), which engage behind the detent bevels (34).

16. Evaporator according to claim 15, **characterized in** that the detent projections (35) project laterally and horizontally from the lid flange (32).

17. Evaporator according to at least one of the preceding claims, **characterized in** that the housing (14) comprises a housing upper part (36) and a housing lower part (37), wherein wall portions (38) of housing upper and housing lower parts (36, 37) which are directed towards one another engage one behind the other.

18. Evaporator according to claim 17, **characterized in** that housing upper and housing lower parts (36, 37) have detent devices (39), which are complementary to one another.

19. Evaporator according to claim 17, **characterized in** that a connector component (40) is supported in the housing upper part (36) and/or in the housing lower part (37) so as to be rotatable through an angle of at least 90°.

20. Evaporator according to claim 19, **characterized in** that the connector component (40) is electrically connected by at least one switch (41) and an indicator lamp (42) to the heating element (5).

## Revendications

1. Evaporateur (1) pour l'évaporation de fluides (2) très volatils, en particulier d'agents insecticides fluides, comprenant un réceptacle (3) destiné au fluide (2), une mèche (4) aspirant le fluide, et un élément chauffant (5) associé à la mèche, caractérisé par le fait que la mèche (4) est placée à distance du réceptacle (3) et est reliée à ce dernier par l'intermédiaire d'un conduit d'alimentation (6), en vue de l'amenée du fluide (2).

2. Evaporateur selon la revendication 1, caractérisé par le fait que la mèche (4) est placée au-dessous du réceptacle (3).

3. Evaporateur selon la revendication 1 ou 2, caractérisé par le fait que la mèche (4) est constituée d'un matériau anorganique poreux.

4. Evaporateur selon la revendication 3, caractérisé par le fait que la mèche (4) est formée d'un corps évaporateur (7) pour l'essentiel rectangulaire.

5. Evaporateur selon la revendication 4, caractérisé par le fait que l'élément chauffant (5) est associé à une face inférieure (8) du corps évaporateur (7) qui est tournée à l'opposé d'une face supérieure (9) délivrant le fluide évaporé (2), et le conduit d'alimentation (6) est raccordé à une surface latérale (10) du corps évaporateur (7).

6. Evaporateur selon la revendication 5, caractérisé par le fait que la surface latérale (10) présente une protubérance (11) pouvant être emboîtée dans une première extrémité (20) du conduit d'alimentation (6).

7. Evaporateur selon la revendication 4, caractérisé par le fait que le corps évaporateur (7) est monté, au moins par sa face inférieure (8), sur une tôle de support (12) thermiquement conductrice sur laquelle l'élément chauffant (5) est placé.

8. Evaporateur selon la revendication 4, caractérisé par le fait que l'élément chauffant (5) est placé sur une face inférieure (13) de la tôle de support (12) qui est tournée à l'opposé du corps évaporateur (7).

9. Evaporateur muni d'un boîtier (14) logeant le réceptacle (3), le corps évaporateur (7), la tôle de support (12) et l'élément chauffant (5), le réceptacle (3) étant fixé amoviblement audit boîtier, selon la revendication 7, caractérisé par le fait que des éléments encliquetables (15) font saillie au-delà du réceptacle (3) et des éléments d'encliquetage complémentaires (16), pouvant coopérer par déclic avec les éléments précités, sont ménagés sur le boîtier (14).

10. Evaporateur selon la revendication 9, caractérisé par le fait que les éléments encliquetables (15) sont réalisés sous la forme de crochets à déclic saillant au-delà d'une face inférieure (17) du réceptacle (3), et les éléments d'encliquetage complémentaires (16) sont des orifices d'encliquetage pratiqués dans une surface réceptrice (18) ménagée sur le boîtier (14) et affectée à la face inférieure (17) du réceptacle.

11. Evaporateur selon la revendication 10, caractérisé par le fait qu'un embout de raccordement (19), sur lequel peut être emboîtée une seconde extrémité (21) du conduit d'alimentation (6), fait saillie au-delà de la face inférieure (17) du réceptacle, un orifice (22), correspondant audit embout de raccordement (19), étant pratiqué dans la surface réceptrice (18).

12. Evaporateur selon la revendication 10, caractérisé par le fait que la surface réceptrice (18) comporte un rebord périphérique (23) qui fait saillie vers le haut en direction du réceptacle (3) et forme, au moins le long d'un côté dudit réceptacle (3), une paroi d'appui (24) présentant une hauteur (25) correspondant pour l'essentiel à une hauteur (26) dudit réceptacle.

13. Evaporateur selon au moins l'une des revendications précédentes, caractérisé par le fait que le réceptacle (3) présente, à son extrémité supérieure (27), un orifice de remplissage (29) entouré par un rebord (28) pour l'essentiel horizontal, un couvercle (30), pouvant être mis en place par coulissement sur l'orifice de remplissage, étant muni d'une rainure de coulissement (31) complémentaire dudit rebord.

14. Evaporateur selon la revendication 13, caractérisé par le fait que le couvercle (30) présente une aile faisant saillie en direction de la paroi d'appui (24) et insérée, lorsque ledit couvercle (30) est mis en place par coulissement sur l'orifice de remplissage (29), dans une perforation (23) ménagée à l'extrémité supérieure de ladite paroi d'appui (24).

15. Evaporateur selon la revendication 14, caractérisé par le fait que deux rampes d'encliquetage (34) dépassent dans la perforation (33), à partir de la paroi d'appui (24), et l'aile (32) du couvercle présente des saillies encliquetables complémentaires (35) emprisonnant lesdites rampes d' encliquetage (34).

16. Evaporateur selon la revendication 15, caractérisé par le fait que les saillies encliquetables (35) dépassent latéralement et horizontalement au-delà de l'aile (32) du couvercle.

17. Evaporateur selon au moins l'une des revendications précédentes, caractérisé par le fait que le boîtier (14) comprend une partie supérieure (36) et une partie inférieure (37), des régions de paroi (38) desdi-tes parties supérieure et inférieure (36, 37) du boîtier, orientées l'une vers l'autre, venant s'emprisonner mutuellement.

18. Evaporateur selon la revendication 17, caractérisé par le fait que les parties supérieure et inférieure (36, 37) du boîtier présentent des systèmes d'encliquetage (39) de réalisations complémentaires.

19. Evaporateur selon la revendication 17, caractérisé par le fait qu'une pièce structurelle d'enfichage (40) est montée à rotation d'un angle d'au moins 90° dans la partie supérieure (36) du boîtier et/ou dans la partie inférieure (37) dudit boîtier.

20. Evaporateur selon la revendication 19, caractérisé par le fait que la pièce structurelle d'enfichage (40) est connectée électriquement à l'élément chauffant (5) par l'intermédiaire d'au moins un interrupteur (41) et d'un témoin lumineux (42).
